# FASCICULE DE BREVET EUROPEEN

(11) **EP 0 337 287 B1**
(45) Date de publication et mention de la délivrance du brevet: **04.10.1995**
(21) Numéro de dépôt: 89106027.9
(22) Date de dépôt: 06.04.1989
(51) Int. Cl.: G01S 15/66, G01S 15/18

(54) **Suiveur d'écho pour appareil de mesure par ultrasons de la position d'une paroi mobile**
Echofolger für ein Ultraschallmessgerät zum Feststellen der Position einer beweglichen Fläche
Echofollower for ultrasonic apparatus for measuring the position of a movable reflective surface

(30) Priorité: 14.04.1988 FR 8805065
(43) Date de publication de la demande: 18.10.1989
(73) Titulaire: ASULAB S.A., CH-2501 Bienne (CH)
(72) Inventeur: Etienne, Jean-Daniel, CH-2006 Neuchâtel (CH); Farine, Pierre-André, CH-2003 Neuchâtel (CH); Bornoz, Claude, CH-2114 Fleurier (CH)
(74) Mandataire: Caron, Gérard

(56) Documents cités:
- FR-A- 2 280 089
- US-A- 3 618 085
- US-A- 4 258 362
- UTRASOUND IN MEDICINE AND BIOLOGY, vol. 8, no. 2, mars 1982, pages 185-190,Pergamon Press, Oxford, GB; D.H. GROVES et al. : "A digital technique for tracking moving interfaces"

## Description

La présente invention concerne un suiveur d'écho pour un appareil de mesure de la position d'une paroi mobile.

L'invention trouve une application dans tous les domaines dans lesquels on souhaite suivre l'évolution temporelle de la position d'une paroi mobile, et notamment dans le domaine médical. Dans ce dernier, l'invention peut être utilisée pour suivre l'évolution temporelle de la position d'une interface entre deux tissus, et par exemple les profondeurs des parois antérieure et postérieure d'un vaisseau sanguin, pour déterminer l'évolution temporelle du diamètre de ce vaisseau sanguin.

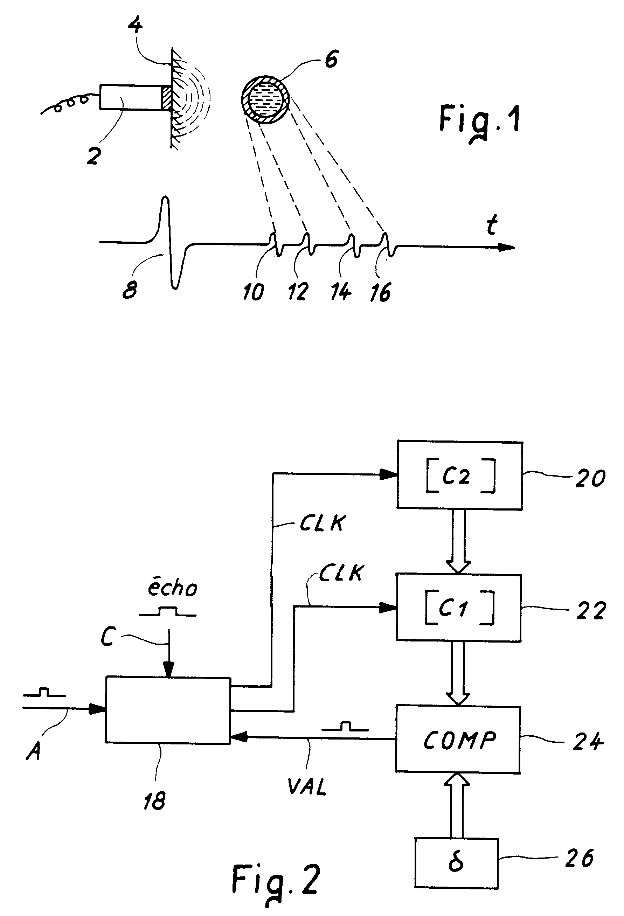

La figure 1 illustre schématiquement le principe connu de la mesure de la position d'une paroi mobile. Cette figure représente un transducteur d'onde ultrasonore 2 placé sur la peau 4 d'un sujet en face de l'artère radiale 6 représentée en coupe transversale. Le transducteur 2 est commandé par un circuit électronique pour émettre une impulsion d'onde ultrasonore 8 et pour recevoir les échos résultant de la réflexion de cette impulsion sur les interfaces artère - tissu ou artère - sang. Selon la fréquence du transducteur ultrasonore, on peut détecter quatre échos distincts 10, 12, 14, 16 ou seulement deux échos correspondant respectivement à une combinaison des échos 10 et 12, et à une combinaison des échos 14 et 16.

On détermine le mouvement d'une interface de la manière suivante. Le transducteur 2 émet une impulsion 8 avec une fréquence de répétition comprise généralement entre 100 Hz et 20 kHz. Pour suivre la position de l'écho, dont le retard sur l'impulsion 8 dépend de la position de l'interface, on utilise une fenêtre temporelle de largeur fixée qui définit un intervalle de temps dans lequel l'écho est attendu, et qui est ouverte avec un retard ajustable après l'emission de impulsion 8. Ce retard est ajusté, après chaque cycle, de manière que l'écho soit détecté au centre de cette fenêtre si l'interface est immobile.

La connaissance de la position de chaque interface en fonction du temps permet, par différence, de déterminer l'évolution du diamètre du vaisseau sanguin 6 en fonction du temps.

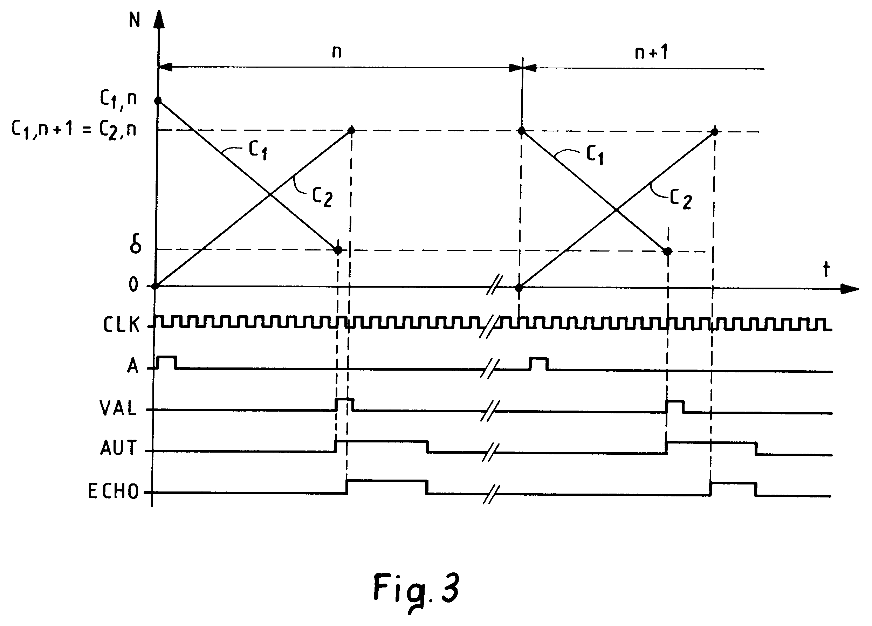

Un suiveur d'écho est décrit notamment dans l'article " A phase-locked echo tracking system for recording arterial diameter changes in vivo " de D. E. Hokanson et al., paru dans Journal of Applied Physiology, vol 32, no 5, pp 728-733, 1972. Ce suiveur d'écho est essentiellement analogique ce qui en limite la précision. Plus récemment, des suiveurs d'écho numériques ont été proposés. Un tel suiveur d'écho est décrit notamment dans l'article " A digital technique for tracking moving interfaces " de D. H. Groves et al, paru dans Ultrasound Med. & Biol., vol 8, no 2, pp 185-190, 1982. On a représenté sur la figure 2 un circuit illustrant la structure de ce suiveur d'écho numérique, et sur la figure 3 un chronogramme illustrant le fonctionnement de ce circuit.

Ce suiveur d'écho comprend un circuit logique 18, un compteur de profondeur 20, un compteur d'autorisation 22, un comparateur numérique 24 et un registre 26. Le circuit logique 18 reçoit un signal d'impulsions A synchrone avec le signal d'excitation appliqué au transducteur, un signal VAL délivré par le comparateur 24 pour indiquer le début de la fenêtre temporelle, et un signal ECHO qui représente l'écho reçu par le transducteur ultrasonore après mise en forme et numérisation. Le circuit logique 18 inclut un générateur produisant un signal d'horloge CLK qui est utilisé pour mesurer le retard de l'écho sur l'impulsion A, et pour rythmer les compteurs 20, 22.

La figure 3 illustre le fonctionnement de ce suiveur d'écho pour deux cycles consécutifs n et n+1. Légèrement avant le début du cycle n, le compteur 22 est chargé avec une valeur C _{1,n} qui, comme on le verra dans dans la suite, est égale au contenu du compteur 20 à la fin du cycle n - 1. Cette valeur est telle que, en décomptant au rythme du signal d'horloge CLK, le contenu du compteur est égal à zéro à l'instant correspondant au milieu de la fenêtre temporelle si la paroi est immobile. Au moment ou la fenêtre temporelle doit être ouverte, le compteur 22 contient donc une valeur non nulle δ. Pour déterminer le début de la fenêtre temporelle, il suffit donc de comparer le contenu du compteur 22 à la valeur fixe δ. Ceci est réalisé par le comparateur 24 qui reçoit d'une part la valeur contenue dans le compteur 22 et d'autre part la valeur δ mémorisée dans le registre 26.

En ce qui concerne le compteur 20, son contenu est mis à zéro peu avant le cycle n. Son comptage est stoppé par le circuit logique 18 sur réception de l'écho C. Le compteur 20 contient alors une valeur C _{2,n} qui représente la position de la paroi mobile. Cette valeur est utilisée dans le cycle suivant n + 1 pour recaler la position de la fenêtre temporelle. Ceci est réalisé en deux étapes : le contenu du compteur 20 est transféré dans le compteur 22, et le compteur 20 est mis à zéro.

On sait que les suiveurs d'écho traitent des signaux numériques de fréquence très élevée. Dans le suiveur d'écho connu représenté sur la figure 2, le transducteur émet à une fréquence de répétition de 12,5 kHz des impulsions à 3,5 MHz, et le signal d'horloge CLK a une fréquence de 20 MHz.

Il s'ensuit que le suiveur d'écho représenté sur la figure 2 présente une consommation électrique élevée car les deux compteurs 20, 22 et le comparateur 24 fonctionnent chacun pendant une grande partie de chaque cycle. Ceci constitue un obstacle à la réalisation d'un appareil portable à cause du poids important de la batterie ou de l'accumulateur nécessaire pour que l'autonomie électrique de l'appareil soit raisonnable.

Or, il est clair qu'un besoin en appareils portables existe dans des domaines tels que le domaine médical. En effet, il serait plus simple à un médecin de transporter l'appareil d'une chambre à une autre dans un hôpital pour examiner les malades plutôt que de devoir déplacer les malades vers un appareil fixe. Un appareil portable permettrait également au médecin d'examiner un malade à son domicile. Enfin, un appareil portable suffisamment petit pourrait être porté directement par l'usager, par exemple sous forme de montre bracelet.

Un autre inconvénient de l'appareil connu représenté sur la figure 2 réside dans son coût élevé, dû en partie à l'utilisation d'un comparateur très rapide. Ceci constitue un frein au développement industriel de ce type d'appareil.

L'invention a pour but une diminution de la consommation électrique et une simplification de l'architecture des suiveurs d'écho pour permettre notamment la réalisation à un prix abordable d'un appareil portable de mesure par ultrasons de la position d'une paroi mobile.

L'invention réside dans une nouvelle structure de suiveur d'écho qui entraîne une réduction importante de la consommation électrique et qui permet de supprimer le comparateur.

De manière précise, l'invention a pour objet un suiveur d'écho pour un appareil de mesure par ultrasons de la position d'une paroi mobile comprenant une entrée pour recevoir un signal numérique d'écho produit par la réflexion sur ladite paroi mobile d'une impulsion d'interrogation dont l'émission est commandée par une impulsion de commande A émise à une fréquence de répétition Fᵣ, ledit suiveur d'écho comprenant une horloge délivrant un signal d'horloge CLK, un moyen d'autorisation délivrant un signal d'autorisation AUT pour définir une fenêtre temporelle dans laquelle l'écho est attendu, un circuit logique pour délivrer une donnée POSREC de position recalée de ladite fenêtre en fonction d'une donnée POSECH de position de l'écho dans la fenêtre, et un compteur de profondeur pour définir le début de ladite fenêtre temporelle, ledit compteur de profondeur comptant à la cadence du signal d'horloge CLK entre ladite donnée de position recalée et une première valeur déterminée, le début du comptage étant commandé par ladite impulsion de commande et le compteur de profondeur émettant un signal de validation VAL lorsqu'il atteint ladite première valeur déterminée pour activer ledit moyen d'autorisation, ledit suiveur d'écho étant caractérisé en ce qu'il comprend en outre :
- un détecteur de position pour déterminer la position de l'écho dans ladite fenêtre temporelle, ledit détecteur de position comptant à la cadence du signal d'horloge CLK depuis une deuxième valeur déterminée pour que ladite donnée de position d'écho soit égale à zéro lorsque la paroi est immobile, le début du comptage étant commandé par ledit signal de validation et ledit détecteur de position émettant vers ledit circuit logique une donnée de position d'écho dans ladite fenêtre temporelle, ledit circuit logique (34) comprenant un moyen (44) pour délivrer la donnée POSREC comme somme algébrique de la donnée POSREC précédente et de la donnée POSECH reçue du détecteur de position (32), si ladite deuxième valeur est négative, ou comme différence algébrique de ces deux données respectivement POSREC et POSECH, si ladite deuxième valeur est positive, et ledit compteur de profondeur comporte une borne dont l'état logique est différent selon que le contenu du compteur est égal à zéro ou est différent de zéro, ladite première valeur déterminée étant choisie égale à zéro et ledit signal de validation est prélevé sur ladite borne.

De manière avantageuse, le moyen d'autorisation peut être un compteur ou un registre à décalage, et le signal d'autorisation peut être prélevé sur une borne du moyen d'autorisation comme le signal de validation est prélevé sur une borne du compteur de profondeur.

Il convient de préciser que les compteurs utilisés dans l'invention servent à mesurer des intervalles de temps et qu'à ce titre le sens de comptage est indifférent. L'invention peut donc être mise en oeuvre aussi bien par des compteurs que par des décompteurs bien que, pour simplifier, le terme de compteur seul soit utilisé dans le texte.

Les caractéristiques et avantages de l'invention ressortiront mieux de la description qui va suivre, donnée à titre illustratif mais non limitatif, en référence aux dessins annexés, sur lesquels :
- la figure 1, déjà décrite, montre de manière schématique le principe de la mesure de la position d'une paroi mobile,
- la figure 2, déjà décrite, représente un suiveur d'écho selon l'art antérieur,
- la figure 3, déjà décrite, est un chronogramme illustrant le fonctionnement du suiveur d'écho de la figure 2,
- la figure 4 illustre un mode de réalisation d'un suiveur d'écho selon l'invention,
- la figure 5 est un chronogramme illustrant le fonctionnement du suiveur d'écho de la figure 4, et
- la figure 6 représente un appareil de mesure de la position d'une paroi mobile comportant deux suiveurs d'écho selon 1' invention.

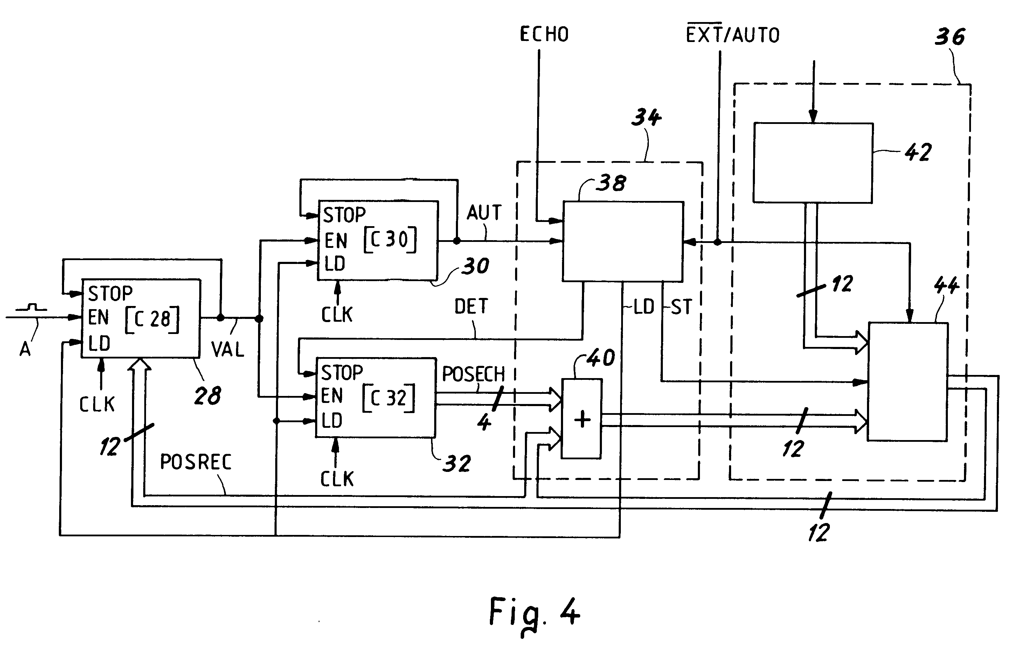

On a représenté sur la figure 4 un mode de réalisation du suiveur d'écho selon l'invention. Ce suiveur d'écho a pour fonction de détecter les échos correspondants à des impulsions ultrasonores émises avec une fréquence de répétition Fᵣ vers une paroi mobile. Pour chaque écho, la détection est autorisée dans une fenêtre temporelle de largeur déterminée dont la position est asservie sur l'instant de détection de l'écho précédant l'écho attendu. A cette fin, le suiveur d'écho comprend un compteur de profondeur 28 pour déterminer l'instant d'ouverture de la fenêtre temporelle, un moyen d'autorisation 30 pour définir la largeur de la fenêtre temporelle, un détecteur de position 32 pour mémoriser la position de l'écho reçu dans la fenêtre temporelle, et un circuit logique 34 pour recaler la fenêtre temporelle en fonction de la position de l'écho indiquée par le détecteur de position.

Le suiveur d'écho représenté sur la figure 4 comprend en outre un moyen de commande externe 36 pour modifier la position de la fenêtre temporelle indépendamment de la position de l'écho reçu. Ce moyen 36 est utilisé en phase d'initialisation ou, plus généralement, à chaque fois que les échos sortent de la fenêtre.

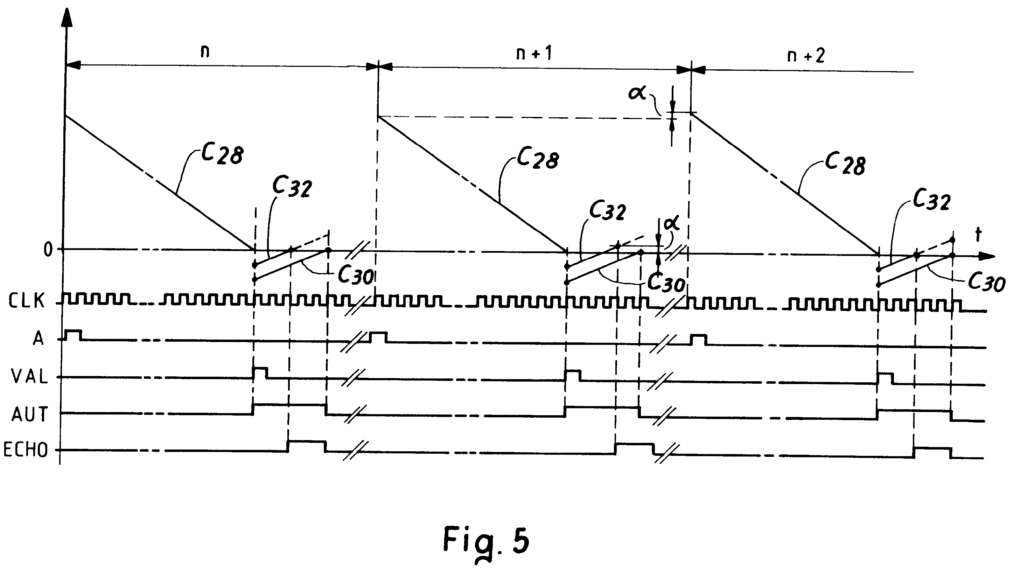

Le chronogramme de la figure 5 illustre le fonctionnement du suiveur d'écho. Celui-ci exécute des cycles successifs n, n+1, n+2... à la fréquence de répétition Fᵣ du signal de commande A. Avant le début de chaque cycle, le circuit logique 34 émet un signal LD pour charger des valeurs déterminées dans le compteur de profondeur 28, le moyen d'autorisation 30 et le détecteur de position 32.

La valeur POSREC chargée dans le compteur de profondeur 28 dépend de la position de l'écho dans la fenêtre. Cette valeur est stockée dans une mémoire 44 qui sera décrite ultérieurement. Les valeurs chargées dans le moyen d'autorisation 30 et le détecteur de position 32 sont des valeurs fixes contenues dans des mémoires non représentées sur la figure 4.

La valeur chargée dans le compteur de profondeur 28 est choisie de manière que ce compteur délivre le signal de validation VAL à l'instant où la fenêtre temporelle doit être ouverte. Ce signal de validation est émis lorsque le compteur contient une première valeur déterminée. De manière préférée, on utilise un compteur de profondeur muni d'une borne de sortie dont l'état logique est différent selon que la valeur contenue dans le compteur est égale à zéro ou est différente de zéro. Le signal de validation est alors obtenu directement sur cette borne de sortie. Le signal de validation peut également être constitué de manière simple par la valeur d'un bit de rang particulier du nombre binaire contenu dans le compteur. Il suffit en effet de charger initialement le compteur de profondeur avec une valeur choisie de manière à ce que le bit de rang sélectionné change d'état à l'instant où l'on désire ouvrir la fenêtre.

Le signal de validation délivré par le compteur de profondeur 28 sert principalement à activer le moyen d'autorisation 30 et le détecteur de position 32. Il peut être appliqué en outre sur une entrée STOP du compteur de profondeur 28 pour en arrêter le comptage.

Sur réception du signal de validation, le moyen d'autorisation 30 délivre un signal d'autorisation AUT de longueur déterminée qui définit la fenêtre temporelle pendant laquelle un écho est attendu. Ce moyen d'autorisation peut être réalisé par un compteur. Dans ce cas le signal d'autorisation peut être produit de la même manière que le signal de validation, c'est-à-dire qu'il peut être constitué par l'état logique d'un bit de rang déterminé du nombre binaire contenu dans le compteur, ou par l'état logique d'un bit ayant une valeur différente selon que le contenu du compteur est égal à zéro ou est différent de zéro.

Un autre mode de réalisation du moyen d'autorisation consiste à utiliser un registre à décalage. Dans ce mode de réalisation, le signal d'autorisation peut également être formé par l'état logique d'un bit de rang déterminé du registre à décalage ou par la valeur d'un bit dont l'état logique est différent selon que le contenu du registre à décalage est égal à zéro ou est différent de zéro.

Par exemple, pour produire un signal d'autorisation à l'état haut pendant 5 périodes du signal d'horloge CLK, on peut choisir pour signal d'autorisation la valeur du bit de poids le plus faible d'un registre à décalage de 8 bits chargé avec la valeur "00011111". En effet, ce bit vaut "1" pendant les 5 premiers décalages à droite commandés par le signal CLK puis passe à "0" à partir du 6ème décalage à droite.

Le détecteur de position 32 est activé par le signal de validation émis par le compteur de profondeur 28. Son comptage est arrêté par un signal de détection délivré par l'unité 38 du circuit logique 34 sur une entrée STOP du détecteur de position 32 à l'instant où le signal d'écho apparaît dans la fenêtre temporelle.

Dans ce mode de réalisation, c'est l'unité logique 38 qui détecte le signal d'écho et qui vérifie qu'il apparait dans la fenêtre temporelle. Cette vérification peut être réalisée simplement par une porte logique ET. Il est clair que l'on pourrait également appliquer le signal d'écho directement sur l'entrée d'arrêt STOP du détecteur d'écho et vérifier ensuite avec l'unité logique si la valeur contenue dans le détecteur de position correspond à un écho reçu dans ou hors de la fenêtre temporelle.

La valeur POSECH contenue dans le détecteur de position 32 représente le déplacement à appliquer à la fenêtre pour la recentrer sur l'écho reçu. Ce déplacement est réalisé par une mise à jour de la valeur chargée par le circuit logique 34 dans le compteur de profondeur 28. Cette mise à jour est particulièrement simple si la valeur initiale chargée dans le détecteur de position 32 au début du cycle est choisie de manière que, dans le cas d'une paroi immobile, le détecteur de position contient la valeur zéro lorsque l'écho est détecté. La valeur chargée dans le compteur de profondeur est alors modifiée simplement en lui ajoutant la valeur contenue dans le détecteur de position 32. Ceci est réalisé par un additionneur 40. Cette valeur de chargement du compteur de profondeur est mémorisée dans une mémoire 44 dont l'accès est autorisé par un signal ST émis par l'unité logique 38.

Le chronogramme de la figure 5 illustre les principaux signaux apparaissant sur la figure 4, ainsi que les valeurs contenues dans les compteurs. Les références C28, C30 et C32 correspondent respectivement aux contenus du compteur de profondeur 28, d'un compteur formant le moyen d'autorisation 30, et du détecteur de position 32.

Chaque cycle de mesure commence avec une impulsion de commande du signal A. Cette impulsion fait débuter le comptage du compteur 28 jusqu'à la première valeur déterminée (égale à zéro sur la figure 5) qui provoque l'émission du signal de validation VAL.

Ce signal active le moyen d'autorisation 30 et le détecteur de position 32. Le signal d'autorisation AUT, qui définit la fenêtre temporelle, s'affirme pendant une durée prédéterminée qui, sur la figure 5, est égale à 5 périodes du signal CLK. Cette durée est définie par la durée d'un comptage jusqu'à une valeur déterminée qui est égale à zéro sur la figure.

A partir du début de la fenêtre temporelle, le contenu C32 du détecteur de position 32 évolue jusqu'à l'apparition de l'écho. La valeur à cet instant reflète la position de l'écho dans la fenêtre, et donc la valeur de recalage de position à appliquer à la fenêtre.

Sur la figure, la valeur chargée dans le détecteur de position 32 est choisie pour que, dans le cas d'une paroi immobile, l'écho soit détecté lorsque le détecteur de position contient la valeur zéro. Ceci permet de recaler très facilement la fenêtre comme le montrent les cycles successifs n, n+1, n+2.

Au cours du cycle n, l'écho apparait au centre de la fenêtre (C32 est égal à zéro). La fenêtre est donc en position correcte et le compteur de profondeur 28 est chargé pour le cycle n+1 avec la même valeur que pour le cycle n. La fenêtre temporelle apparait donc au même instant, par rapport au début du cycle défini par l'impulsion de commande A, au cours des cycles n et n+1.

Dans le cycle n+1, l'écho apparait plus tard dans la fenêtre, montrant que la paroi mobile s'est éloignée de l'appareil de mesure. Ce retard se traduit par une valeur α supérieure à zéro dans le détecteur de position. Pour recaler la fenêtre, cette valeur α est ajoutée à la valeur chargée dans le compteur de profondeur 28 pour le cycle n+2.

Si au contraire la paroi mobile se rapproche de l'appareil de mesure, l'écho apparait avant le milieu de la fenêtre et le détecteur de position contient alors une valeur négative. Il suffit également d'ajouter cette valeur négative pour recentrer la fenêtre. Bien entendu, on peut remplacer le compteur 32 par un décompteur et, dans ce cas, l'additionneur est remplacé par un soustracteur.

Sur la figure 5, on note que les signaux A et CLK sont indépendants. Ceci n'est pas nécessaire pour le fonctionnement du suiveur d'écho mais permet, en aval, de faire un moyennage sur les valeurs de positions de la paroi mobile. Il en résulte une augmentation de la résolution comme on le verra dans la suite de la description.

En mode de fonctionnement normal, la position de la fenêtre est recalée automatiquement en fonction de la position des échos. Cependant, il est possible que, de manière accidentelle, la fenêtre ne soit plus calée sur le signal d'écho, par exemple si le signal d'écho disparait pendant un certain temps et que la paroi mobile se déplace. Par ailleurs, à la mise en marche de l'appareil, l'écho n'apparait pas nécessairement dans la fenêtre. Pour ces raisons, le suiveur d'écho représenté sur la figure 4 est pourvu d'un moyen de commande externe de la position de la fenêtre.

Ce moyen de commande externe 36 comprend un compteur 42 et une mémoire 44 à deux entrées multiplexées. La valeur contenue dans le compteur 42 peut être modifiée par un signal externe de commande de comptage ou de décomptage. La valeur du compteur 42 est appliquée sur une entrée de la mémoire 44. L'autre entrée de cette mémoire reçoit la valeur délivrée par l'additionneur 40.

La sélection entre le mode manuel et le mode automatique de calage de la fenêtre est défini par un signal externe EXT/AUTO qui sélectionne l'une des entrées de la mémoire 44. Ce signal est également appliqué à l'unité logique 38 qui délivre le signal ST d'accès à la mémoire 44.

A titre d'exemple, un suiveur d'écho conforme à la figure 4 et fonctionnant avec une horloge CLK à 60 MHz peut comprendre des compteurs à 12 bits de type 74ACT191 pour le compteur de profondeur 28, le moyen d'autorisation 30, le détecteur de position 32 et le compteur 42, un additionneur de type 74HCT283 pour l'additionneur 40, et un ensemble comprenant une mémoire de type 74HCT174 et un multiplexeur de type 74HCT604 pour la mémoire 44. En ce qui concerne l'unité logique 38, on peut utiliser un ensemble de portes logiques et de bascules bistables bien connues de spécialistes.

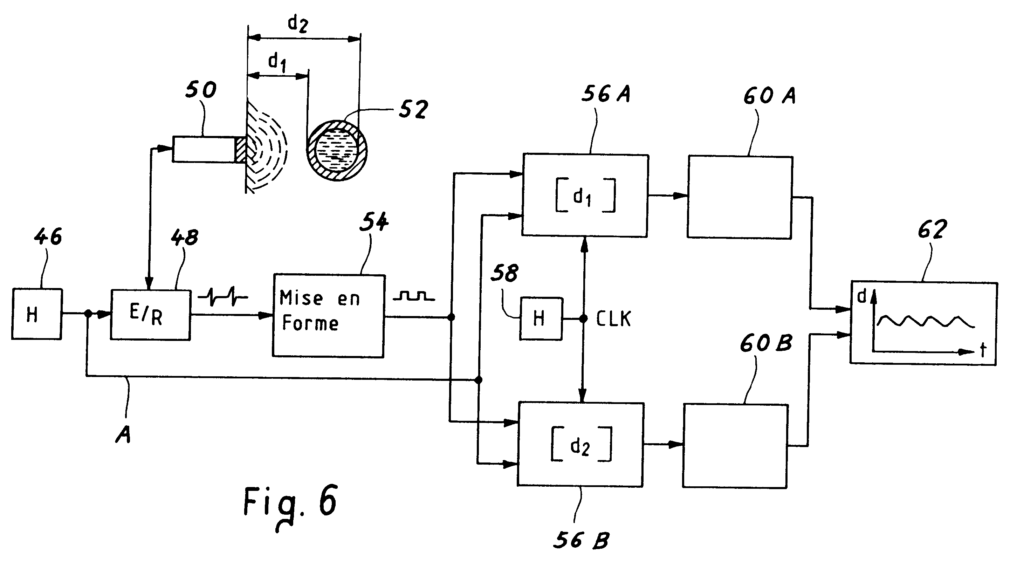

Le suiveur d'écho qui vient d'être décrit est un élément d'un appareil de mesure par ultrasons de la position d'une paroi mobile. Il a une structure plus simple et présente une consommation électrique moins élevée que le suiveur d'écho connu représenté sur la figure 2. Le suiveur d'écho selon l'invention peut ainsi être intégré dans un appareil de mesure portable. On a représenté schématiquement sur la figure 6 un mode de réalisation d'un tel appareil, qui est appliqué dans le domaine médical à la mesure du diamètre d'une artère.

Cet appareil comprend une horloge 46 délivrant un signal d'impulsion à une fréquence de répétition de 5 kHz. Ce signal est reçu par un circuit d'émission-réception 48 qui commande un transducteur ultrasonore 50. Celui-ci émet une impulsion de fréquence 10 MHz vers l'artère 52 et recueille les échos réfléchis par les parois antérieure et postérieure de cette artère. Ces échos sont délivrés par le circuit 48 à un moyen de mise en forme 54 comprenant de manière connue des moyens de filtrage et un convertisseur analogique - numérique.

Le signal numérique délivré par le moyen de mise en forme 54 est appliqué sur les entrées de deux suiveurs d'échos 56A, 56B qui sont chacun conformes au suiveur d'écho représenté sur la figure 4. Les suiveurs d'échos reçoivent un même signal d'horloge CLK de fréquence 60 MHz délivré par une horloge 58. En pratique, chaque suiveur d'écho peut également être réalisé sur une carte indépendante comprenant une horloge CLK. Dans ce cas, chaque suiveur d'écho possède sa propre horloge. Ces horloges n'ont pas besoin d'être synchronisées.

Les fenêtres temporelles des suiveurs d'échos 56A et 56B sont calés respectivement sur l'écho produit par la paroi antérieure et l'écho produit par la paroi postérieure de l'artère 52. Chaque suiveur d'écho délivre donc, à la fréquence de répétition Fᵣ, la position de la paroi de l'artère sur laquelle il est calé, c'est-à-dire la distance entre cette paroi et le transducteur 50.

La suite des positions délivrée par chaque suiveur d'écho est reçue dans un circuit de moyennage 60A, 60B. Chaque circuit permet, de manière connue, d'augmenter la précision de la mesure en calculant la valeur moyenne d'un ensemble de valeurs de positions consécutives ou en faisant une moyenne glissante sur un ensemble de valeurs de position consécutives.

On sait en effet que la résolution brute ε_{b} sur la position d'une paroi mobile est égale à c/ (2. F_{CLK}), où c est la vitesse de propagation du son dans le milieu situé entre le transducteur et la paroi, et F_{CLK} la fréquence du signal CLK, alors que la résolution effective εₑ après moyennage sur des ensembles de N positions consécutives est égale à ε_{b}/N. Cette opération de moyennage n'est possible que si les horloges 46 et 58 sont indépendantes.

Les valeurs délivrées par les circuits de moyennage 60A, 60B sont reçues dans un circuit 62 d'exploitation des résultats. Ce circuit peut comprendre une unité de mémorisation et une unité d'affichage. Il peut comprendre également une unité de calcul pour calculer le diamètre de l'artère 52 au cours du temps par différence des positions des parois antérieure et postérieure.

De manière avantageuse, le circuit 62 peut comprendre un microordinateur. Dans ce cas, le moyennage peut être réalisé aisément par logiciel.

## Revendications

1. Suiveur d'écho pour appareil de mesure par ultrasons de la position d'une paroi mobile comprenant une entrée pour recevoir un signal numérisé d'écho produit par la réflexion sur ladite paroi mobile d'une impulsion ultrasonore d'interrogation dont l'émission est commandée par une impulsion de commande A émise à une fréquence de répétition Fᵣ, ledit suiveur d'écho comprenant une horloge délivrant un signal d'horloge CLK, un moyen d'autorisation (30) délivrant sur réception d'un signal de validation VAL un signal d'autorisation AUT pour définir une fenêtre temporelle dans laquelle l'écho est attendu, un circuit logique (34) pour délivrer, à la fréquence de répétition Fᵣ, une donnée POSREC de position recalée de ladite fenêtre en fonction d'une donnée POSECH de position de l'écho dans la fenêtre,
et un compteur de profondeur (28) pour définir le début de ladite fenêtre temporelle, ledit compteur de profondeur comptant, à la cadence du signal d'horloge CLK, entre ladite donnée de position recalée POSREC et une première valeur déterminée, le début du comptage étant commandé par ladite impulsion de commande et le compteur de profondeur émettant ledit signal de validation lorsqu'il atteint ladite première valeur déterminée, ledit suiveur d'écho étant caractérisé en ce qu'il comprend en outre :
- un détecteur de position (32) pour déterminer la position de l'écho dans ladite fenêtre temporelle, ledit détecteur de position comptant à la cadence du signal d'horloge CLK, depuis une deuxième valeur déterminée pour que ladite donnée de position d'écho soit égale à zéro lorsque la paroi est immobile, le début du comptage étant commandé par ledit signal de validation et ledit détecteur de position délivrant audit circuit logique ladite donnée de position d'écho POSECH représentative de la position de l'écho reçu dans ladite fenêtre temporelle, ledit circuit logique (34) comprenant un moyen (44) pour délivrer la donnée POSREC comme somme algébrique de la donnée POSREC précédente et de la donnée POSECH reçue du détecteur de position (32), si ladite deuxième valeur est négative, ou comme différence algébrique de ces deux données respectivement POSREC ET POSECH, si ladite deuxième valeur est positive, et ledit compteur de profondeur comporte une borne dont l'état logique est différent selon que le contenu du compteur est égal à zéro ou est différent de zéro, ladite première valeur déterminée étant choisie égale à zéro et ledit signal de validation est prélevé sur ladite borne.

2. Suiveur d'écho selon la revendication 1, caractérisé en ce que le moyen d'autorisation (30) est un compteur.

3. Suiveur d'écho selon la revendication 1, caractérisé en ce que le moyen d'autorisation (30) est un registre à décalage.

4. Suiveur d'écho selon l'une quelconque des revendications 2 et 3 dans lequel le moyen d'autorisation (30) comporte une borne dont l'état logique est différent selon que le contenu du compteur est égal à zéro ou est différent de zéro, caractérisé en ce que ledit signal d'autorisation est prélevé sur ladite borne.

5. Suiveur d'écho selon l'une quelconque des revendications 2 et 3, caractérisé en ce que le signal d'autorisation est égal à l'état logique d'un bit de rang déterminé r₂ de la donnée binaire contenue dans le moyen d'autorisation, ledit moyen d'autorisation étant chargé par ledit circuit logique avec une troisième valeur déterminée pour que ledit bit change d'état à l'instant où la fenêtre doit être fermée.

6. Suiveur d'écho selon la revendication 1, caractérisé en ce qu'il comprend un moyen externe (36) de déplacement de la fenêtre temporelle, ledit moyen externe délivrant une donnée de position recalée ajustable par un signal de commande externe.

7. Suiveur d'écho selon la revendication 6, caractérisé en ce que ledit moyen externe (36) comprend un compteur (42) dont le contenu constitue ladite donnée de position recalée, le signal de commande externe agissant sur ledit compteur pour modifier ladite donnée de position recalée.

8. Suiveur d'écho selon l'une quelconque des revendications 1 à 7, caractérisé en ce que le signal d'horloge CLK et le signal de commande de fréquence Fᵣ sont indépendants.

## Patentansprüche

1. Echofolger für ein Ultraschallmeßgerät zum Feststellen der Position einer beweglichen Fläche, umfassend einen Eingang zum Empfang eines numerisierten Echosignals, erzeugt durch die Reflexion eines Ultraschallabfrageimpulses an der beweglichen Fläche, dessen Aussendung gesteuert wird durch einen Befehlsimpuls A, ausgesandt mit einer Wiederholungsfrequenz Fᵣ, welcher Echofolger einen Taktgeber umfaßt, der ein Taktsignal CLK erzeugt, ein Freigabemittel (30), das bei Empfang eines Validierungssignals VAL ein Freigabesignal AUT abgibt zum Definieren eines Zeitfensters, in dem das Echo erwartet wird, einen Logikschaltkreis (34) zum Abgeben, mit der Wiederholungsfrequenz Fᵣ, einer Größe POSREC der korrigierten Position des Fensters in Abhängigkeit von einer Größe POSECH der Echoposition in dem Fenster,
und einen Tiefenzähler (28) zum Definieren des Beginns des Zeitfensters, welcher Tiefenzähler, mit der Kadenz des Taktsignals CLK, zwischen der korrigierten Positionsgröße POSREC und einem ersten bestimmten Wert zählt, wobei der Beginn der Zählung ausgelöst wird durch den Auslöseimpuls und der Tiefenzähler das Validierungssignal abgibt, wenn er einen ersten bestimmten Wert erreicht, welcher Echofolger dadurch gekennzeichnet ist, daß er ferner umfaßt:
- einen Positionsdetektor (32) zum Bestimmen der Position des Echos in dem Zeitfenster, welcher Positionsdetektor mit der Kadenz des Taktsignals CLK zählt, ausgehend von einem zweiten bestimmten Wert, damit die Echopositionsgröße gleich null wird, wenn die Fläche unbeweglich ist, wobei der Beginn der Zählung ausgelöst wird durch das Validierungssignal und der Positionsdetektor dem Logikschaltkreis die Echopositionsgröße POSECH liefert, die repräsentativ ist für die Position des empfangenen Echos in dem Zeitfenster, welcher Logikschaltkreis (34) ein Mittel (44) umfaßt zum Abgeben der Größe POSREC als algebraische Summe der vorhergehenden Größe POSREC und der Größe POSECH, empfangen von dem Positionsdetektor (32), wenn der zweite Wert negativ ist, oder als algebraische Differenz dieser beiden Größen POSREC bzw. POSECH, wenn der zweite Wert positiv ist, und wobei der Tiefenzähler eine Klemme umfaßt, deren Logikzustand unterschiedlich ist, je nachdem, ob der Inhalt des Zählers gleich null oder von null verschieden ist, welcher erste bestimmte Wert gleich null gewählt wird und das Validierungssignal an der Klemme abgenommen wird.

2. Echofolger nach Anspruch 1, dadurch gekennzeichnet, daß das Freigabemittel (30) ein Zähler ist.

3. Echofolger nach Anspruch 1, dadurch gekennzeichnet, daß das Freigabemittel (30) ein Schieberegister ist.

4. Echofolger nach einem der Ansprüche 2 und 3, bei dem das Freigabemittel (30) eine Klemme aufweist, deren Logikzustand unterschiedlich ist, je nachdem, ob der Inhalt des Zählers gleich null ist oder unterschiedlich von null ist, dadurch gekennzeichnet, daß das Freigabesignal an der genannten Klemme abgenommen wird.

5. Echofolger nach einem der Ansprüche 2 und 3, dadurch gekennzeichnet, daß das Freigabesignal gleich dem Logikzustand eines Bits bestimmten Ranges r₂ ist der binären Größe, enthalten in dem Freigabemittel, welches Freigabemittel durch den Logikschaltkreis mit einem dritten bestimmten Wert geladen wird, damit das Bit seinen Zustand im Zeitpunkt ändert, wo das Fenster geschlossen werden muß.

6. Echofolger nach Anspruch 1, dadurch gekennzeichnet, daß er ein äußeres Mittel (36) für die Verlagerung des Zeitfensters umfaßt, welches äußere Mittel eine korrigierte Positionsgröße liefert, einstellbar durch ein äußeres Steuersignal.

7. Echofolger nach Anspruch 6, dadurch gekennzeichnet, daß das äußere Mittel (36) einen Zähler (42) umfaßt, dessen Inhalt die korrigierte Positionsgröße bildet, wobei das externe Steuersignal auf den Zähler einwirkt zum Modifizieren der korrigierten Positionsgröße.

8. Echofolger nach einem der Ansprüche 1 bis 7, dadurch gekennzeichnet, daß das Taktsignal CLK und das Frequenzsteuersignal Fᵣ unabhängig sind.

## Claims

1. Echo tracking system for apparatus for ultrasonic measurement of the position of a mobile wall, comprising an input to receive a digital echo signal produced by reflection from said mobile wall of an interrogating ultrasonic pulse the transmission of which is controlled by a control pulse A transmitted at a repetition rate Fᵣ, said echo tracking system comprising a clock delivering a clock signal CLK, enabling means (30) delivering, on reception of a validation signal VAL, an enabling signal AUT to define a time-window during which the echo is expected and a logic circuit (34) to deliver, at the repetition rate Fᵣ, a recentred position signal POSREC of said window which is a function of the echo position signal POSECH of the window,
- and a depth counter (28) to define the beginning of said time window, said depth counter counting at the frequency of the clock signal CLK, between said recentred position signal POSREC and a first given value, the beginning of the counting being triggered by said control pulse and the depth counter emitting said validation signal when it reaches said first given value, said echo tracking system being characterized in that it further comprises :
- a position detector (32) to determine the position of the echo in said time window, said position detector counting at the frequency of the clock signal CLK from a second given value so that said position echo signal is equal to zero when the wall is stationary, the beginning of counting being triggered by said validation signal and said position detector delivering to said logic circuit, said echo position signal POSECH representing the echo position received in said time window, said logic circuit (34) comprising means (44) for delivering a signal POSREC represented by the algebraic sum of the preceding signal POSREC and the signal POSECH received from the position detector (32), if said second value is negative, or represented by the algebraic difference of these two signals respectively POSREC and POSECH, if said second value is positive, and said depth counter includes a terminal of which the logic state is different depending on whether the counter content is equal to or different from zero, said first given value being chosen as being equal to zero and said validation signal is measured at said terminal.

2. Echo tracking system according to claim 1, characterized in that the enabling means (30) is a counter.

3. Echo tracking system according to claim 1, characterized in that the enabling means (30) is a shift register.

4. Echo tracking system according to any one of claims 2 and 3 in which the enabling means (30) comprises a terminal of which the logic state is different depending on whether the content of the counter is equal to or different from zero, characterized in that said enabling signal is measured at said terminal.

5. Echo tracking system according to any one of claims 2 and 3, characterized in that the enabling signal is equal to the logic state of a bit of given weight r₂ of the binary signal contained in the enabling means, said enabling means being loaded by said logic circuit with a third given value so that said bit changes state at the moment when the window is to be closed.

6. Echo tracking system according to claim 1, characterized in that it comprises external means (36) for displacing the time window, said external means delivering an adjustable recentred position by an external control signal.

7. Echo tracking system according to claim 6, characterized in that said external means (36) comprises a counter (42) whose content constitutes said recentred position signal, the external control signal acting on said counter to modify said recentred position signal.

8. Echo tracking system according to any one of claims 1 to 7, characterized in that the clock signal CLK and the repetition control signal Fᵣ are independent.
